(19) Europäisches Patentamt
European Patent Office
Office européen des brevets

(11) Veröffentlichungsnummer: **0 136 640**
**A2**

(12) # EUROPÄISCHE PATENTANMELDUNG

(21) Anmeldenummer: 84111348.3

(22) Anmeldetag: 24.09.84

(51) Int. Cl.⁴: C 07 D 213/53
C 07 D 333/22, C 07 D 307/52
C 07 D 211/16, C 07 D 295/18
A 01 N 43/40, A 01 N 43/84
A 01 N 43/08, A 01 N 43/10

(30) Priorität: 04.10.83 JP 184476/83

(43) Veröffentlichungstag der Anmeldung: 10.04.85 Patentblatt 85/15

(84) Benannte Vertragsstaaten: BE CH DE FR GB IT LI

(71) Anmelder: NIHON TOKUSHU NOYAKU SEIZO K.K.
No.4, 2-chome, Nihonbashi Honcho Chuo-ku
Tokyo 103(JP)

(72) Erfinder: Goto, Toshio
20-1-304, 5-chome, Seishin
Sagamihara-shi Kanagawa(JP)

(72) Erfinder: Sakawa, Shinji
1-14-13, Tamadaira
Hino-shi Tokyo(JP)

(74) Vertreter: Schumacher, Günter, Dr. et al,
c/o Bayer AG Konzernverwaltung RP Patentabteilung
D-5090 Leverkusen 1 Bayerwerk(DE)

(54) **Trichloroacryloyloxim-Derivate, Verfahren zu ihrer Herstellung und landwirtschaftliches Fungizid.**

(57) Trichloroacryloyloxim-Derivate der Formel (I)

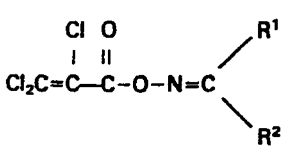

(I)

in der

$R^1$ ein Wasserstoff-Atom, eine niedere Alkyl-Gruppe, eine Phenyl-Gruppe oder eine 2-Pyridyl-Gruppe bezeichnet und

$R^2$ eine, gegebenenfalls durch Methyl substituierte, monocyclische heterocyclische Gruppe mit wenigstens einem Hetero-Atom bezeichnet,

sind als landwirtschaftliche Fungizide wertvoll. Die neuen Trichloroacryloyloxim-Derivate können durch Reaktion eines entsprechenden Acylhalogenids bzw. Acylanhydrids mit einem geeigneten Oxim hergestellt werden.

EP 0 136 640 A2

# Trichloroacryloyloxim-Derivate, Verfahren zu ihrer Herstellung und landwirtschaftliches Fungizid

Die vorliegende Erfindung betrifft ein neues Trichloroacryloyloxim-Derivat, ein Verfahren zu seiner Herstellung und ein landwirtschaftliches Fungizid.
Es ist bereits bekannt, daß bestimmte Trichloroacryloyloxim-Derivate wie beispielsweise O-Trichloroacryloyl-4-(α-chloro-formaldoxim)-pyridin fungizide Eigenschaften besitzen (US-A 4 244 959).

Insbesondere betrifft die vorliegende Erfindung Trichloroacryloyloxim-Derivate der allgemeinen Formel (I)

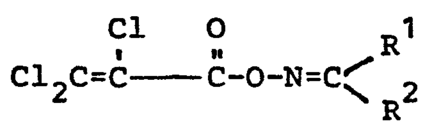

(I)

,

in der

$R^1$ ein Wasserstoff-Atom, eine niedere Alkyl-Gruppe, eine Phenyl-Gruppe oder eine 2-Pyridyl-Gruppe bezeichnet und

$R^2$ eine monocyclische heterocyclische Gruppe mit wenigstens einem Hetero-Atom ausgewählt aus Sauerstoff, Schwefel und Stickstoff bezeichnet, wobei der Hetero-Ring durch eine Methyl-Gruppe substituiert sein kann.

Die Verbindungen der vorstehenden Formel (I) gemäß der vorliegenden Erfindung können beispielsweise mittels des folgenden Verfahrens hergestellt werden, auf das sich die vorliegende Erfindung ebenfalls erstreckt.

Ein Verfahren zur Herstellung der Trichloroacryloyloxim-Derivate der allgemeinen Formel (I) umfaßt die Umsetzung eines Acylhalogenids der allgemeinen Formel (II)

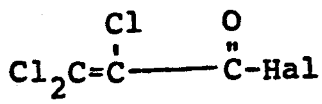

(II)

,

in der
Hal ein Halogen-Atom bezeichnet,
mit einem Oxim der allgemeinen Formel (III)

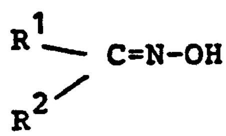

(III)

,

in der
$R^1$ und $R^2$ die oben angegebenen Bedeutungen haben.

Die vorliegende Erfindung betrifft außerdem ein landwirtschaftliches Fungizid, das ein Trichloroacryloyloxim-Derivat der allgemeinen Formel (I) als Wirkstoff enthält.

Seitens der Anmelderin wurden fortgesetzt Untersuchungen durchgeführt, um neue Verbindungen mit biologischer Aktivität zu schaffen. Diese Untersuchungen haben ergeben, daß die bisher in der bekannten Literatur nicht beschriebenen Trichloroacryloyloxim-Derivate der allgemeinen Formel (I) synthetisiert werden können und daß die Verbindungen der Formel (I) ausgezeichnete fungizide Aktivität besitzen.

Erfindungsgemäß wurde gefunden, daß die durch die vorliegende Erfindung bereitgestellten Verbindungen der allgemeinen Formel (I) neue Verbindungen sind, die vor dem Anmeldetag der vorliegenden Anmeldung in bekannten Veröffentlichungen nicht beschrieben worden sind, daß diese Verbindungen in einfacher Weise industriell hergestellt werden können und daß sie eine ausgeprägt gute Wirkung als landwirtschaftliche Fungizide, insbesondere eine Bekämpfungswirkung gegen die Brusone-Krankheit von Reis, aufweisen.

Die Verbindungen der vorliegenden Erfindung sind durch die Tatsache gekennzeichnet, daß in ihrer durch die allgemeine Formel (I) dargestellten chemischen Struktur Trichloroacrylsäure und das oben definierte Oxim vermittels einer Ester-Bindung miteinander verbunden sind. Es wurde gefunden, daß sie aufgrund dieser genannten Struktur eine besonders gute fungizide Aktivität aufweisen. Eine solche fungizide Aktivität läßt sich durch die Anwendung als landwirtschaftliche Fungizide in herkömmlicher Weise nutzen und eignet sich in besonderer Weise für die Bekämpfung der Brusone-Krankheit von Reis.

Es wurde außerdem gefunden, daß die Verbindungen der vorliegenden Erfindung, die neben der oben bezeichneten Aktivität ein ihrer Strukturcharakteristik zuzuschreibendes hervorragendes Eindringvermögen besitzen, eine ausgeprägte Bekämpfungswirkung gegenüber der Brusone-Krankheit von Reis insbesondere dann entfalten, wenn sie auf die Oberfläche oder in das Innere des Wassers auf einem Reisfeld gebracht werden, und infolgedessen in vorteilhafter Weise zur Sicherung des landwirtschaftlichen Arbeitsaufwandes eingesetzt werden können.

Weiterhin wurde gefunden, daß sich die Verbindungen der vorliegenden Erfindung, die niedrige Toxizität gegenüber warmblütigen Tieren aufweisen und in den üblichen Konzentrationen keinerlei Phytotoxizizät gegen Kulturpflanzen zeigen, auch mit einem hohen Maß an Sicherheit einsetzen lassen.

Dementsprechend ist es ein Ziel der vorliegenden Erfindung, neue Trichloroacryloyloxim-Derivate, ein Verfahren zu ihrer Herstellung sowie ihre Verwendung als landwirtschaftliche Fungizide verfügbar zu machen.

Diese und weitere Ziele der vorliegenden Erfindung sowie ihre Vorteile werden im einzelnen aufgrund der folgenden Beschreibung deutlich.

Die Verbindungen der vorliegenden Erfindung haben ein breites fungizides Spektrum und können in wirksamer Weise gegen verschiedene Pflanzenkrankheiten verwendet werden, die beispielsweise durch Archimycetes, Phycomycetes, Ascomycetes, Basidiomycetes und Fungi imperfecti sowie Bakterien verursacht werden.

Die Verbindungen der vorliegenden Erfindung können beispielsweise mittels des folgenden Verfahrens hergestellt werden.

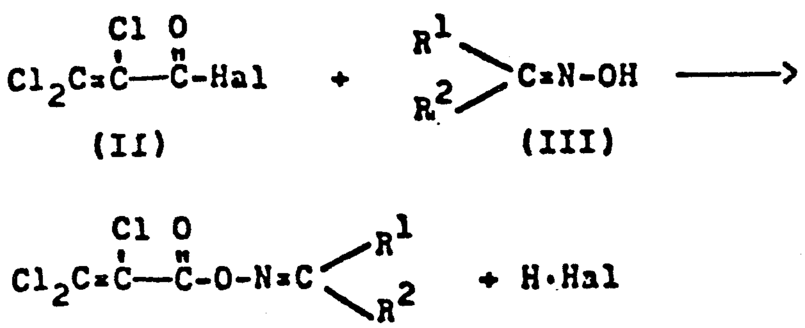

(II) (III)

In dem vorstehenden Reaktionsschema bezeichnen

$R^1$ ein Wasserstoff-Atom, eine niedere Alkyl-Gruppe wie Methyl, Ethyl, Propyl, Isopropyl oder n-(iso-, sec- oder tert-)Butyl, eine Phenyl-Gruppe oder eine 2-Pyridyl-Gruppe und

$R^2$ eine monocyclische heterocyclische Gruppe mit wenigstens einem Hetero-Atom ausgewählt aus Sauerstoff, Schwefel und Stickstoff. Spezielle Beispiele für diese Gruppe sind 2-Pyridyl-, 3-Pyridyl-, 4-Pyridyl-, Furanyl, 3-Thienyl-, 2-Thienyl-, Morpholino- und Piperidino-Gruppen. Die heterocyclische Gruppe kann durch eine Methyl-Gruppe substituiert sein.

Hal bezeichnet speziell solche Halogen-Atome wie Chlor-, Brom-, Iod- und Fluor-Atome und ist vorzugsweise ein Chlor- oder Brom-Atom.

In dem Verfahren zur Herstellung der Verbindungen der vorliegenden Erfindung nach dem obigen Reaktionsschema sind spezielle Beispiele für das Acylhalogenid der allgemeinen Formel (II) als einen der Ausgangsstoffe Trichloroacryloylchlorid und Trichloroacryloylbromid.

Zu speziellen Beispielen für das Oxim der allgemeinen Formel (III) als den anderen Ausgangsstoff zählen Pyridin-2-aldoxim, Pyridin-4-aldoxim, Pyridin-3-aldoxim, Thiophen-3-aldoxim, 5-Methylfuran-2-aldoxim, Methyl-2-thienylketoxim, Methyl-2-pyridylketoxim, Methyl-3-pyridylketoxim, Methyl-4-pyridylketoxim, Phenyl-3-pyridylketoxim, Phenyl-4-pyridylketoxim, Di-2-pyridylketoxim, Phenyl-morpholinoketoxim, Phenyl-4-methylpiperidinoketoxim und 6-Methylpyridin-2-aldoxim.

Das vorstehende Verfahren wird speziell durch das folgende Beispiel beschrieben:

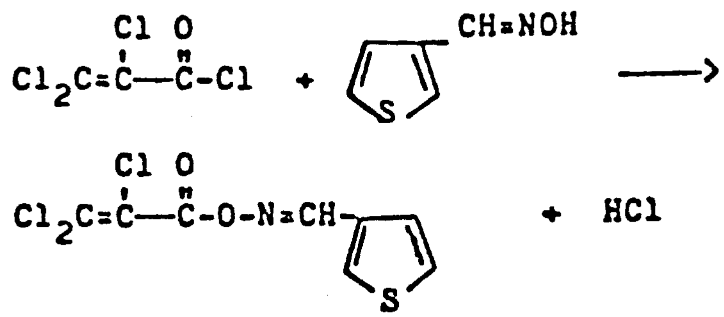

Zweckmäßigerweise kann das Verfahren zur Herstellung der vorstehenden Verbindung gemäß der vorliegenden Erfindung unter Verwendung eines Lösungsmittels oder Verdünnungsmittels durchgeführt werden. Zu diesem Zweck können sämtliche inerten Lösungsmittel und Verdünnungsmittel verwendet werden.

Zu Beispielen für solche Lösungsmittel oder Verdünnungsmittel zählen aliphatische, alicyclische und aromatische Kohlenwasserstoffe (ggf. chloriert) wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol; Ether wie Diethylether, Methylethylether, Diisopropylether, Dibutylether, Propylenoxid, Dioxan und Tetrahydrofuran; Ketone wie Aceton, Methylethylketon, Methylisopropylketon und Methylisobutylketon; Nitrile wie Acetonitril, Propionitril und Acrylnitril; Ester wie Ethylacetat und Amylacetat; Säureamide wie Dimethylformamid und Dimethylacetamid; Sulfone und Sulfoxide wie Dimethylsulfoxid und Sulfolan; und Basen wie Pyridin.

Die obige Reaktion kann in Gegenwart eines säurebindenden Mittels durchgeführt werden. Beispiele für das säurebindende Mittel sind Alkalimetallhydroxide, -carbonate, -hydrogencarbonate und -alkoholate, die allgemein verwendet werden, und tertiäre Amine wie Triethylamin, Diethylanilin und Pyridin.

Das Verfahren gemäß der vorliegenden Erfindung kann in einem weiten Temperaturbereich durchgeführt werden. Beispielsweise kann es bei einer Temperatur zwischen etwa -20°C und dem Siedepunkt der Mischung, vorzugsweise zwischen etwa 0°C und etwa 100°C, durchgeführt werden. Die Reaktion wird zweckmäßigerweise unter Atmosphärendruck durchgeführt. Es ist jedoch auch möglich, unter erhöhtem oder vermindertem Druck zu arbeiten.

Alternativ können die Verbindungen der vorliegenden Erfindung in einfacher Weise auch mittels eines Verfahrens hergestellt werden, das durch das nachstehende Reaktionsschema dargestellt ist.

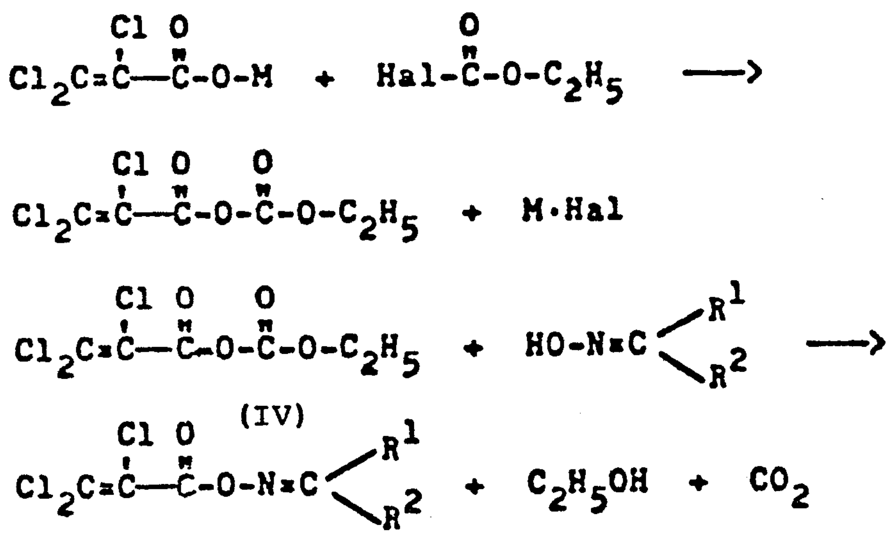

(IV)

(I)

In dem vorstehenden Reaktionsschema steht M für ein Alkalimetall-Atom und $R^1$, $R^2$ und Hal haben die oben angegebenen Bedeutungen.

Als landwirtschaftliche Fungizide können die Verbindungen gemäß der vorliegenden Erfindung unmittelbar nach Verdünnen mit Wasser oder aber in Form verschiedener Formulierungen eingesetzt werden, wie sie unter Verwendung landwirtschaftlich unbedenklicher Hilfsstoffe durch Verfahren gewonnen werden, die bei der praktischen Herstellung von Agrochemikalien allgemein angewandt werden. Im praktischen Einsatz werden diese Präparate entweder unmittelbar oder nach dem Verdünnen mit Wasser auf die gewünschte Konzentration zur Anwendung gebracht.

Beispiele für die landwirtschaftlich unbedenklichen Hilfsstoffe, auf die hier Bezug genommen wird, umfassen Verdünnungsmittel (Lösungsmittel, Streckmittel, Träger), oberflächenaktive Mittel (Lösungsvermittler, Emulgatoren, Dispergiermittel, Netzmittel), Stabilisatoren, Haftmittel, Aerosol-Treibmittel und synergistische Mittel.

Beispiele für die Lösungsmittel sind Wasser und organische Lösungsmittel, beispielsweise Kohlenwasserstoffe /¯z.B. n-Hexan, Petrolether, Naphtha, Erdöl-Fraktionen (z.B. Paraffinwachse, Kerosin, Leichtöle, Mittelöle und Schweröle), Benzol, Toluol und Xylole_7, halogenierte Kohlenwasserstoffe (z.B. Methylenchlorid, Kohlenstofftetrachlorid, Trichloroethylen, Ethylenchlorid, Ethylendibromid, Chlorbenzol und Chloroform), Alkohole (z.B. Methylalkohol, Ethylalkohol, Propylalkohol und

Ethylenglycol), Ether (z.B. Diethylether, Ethylenoxid und Dioxan), Alkohol-ether (z.B. Ethylenglycol-monomethylether), Ketone (z.B. Aceton und Isophoron), Ester (z.B. Ethylacetat und Amylacetat), Amide (z.B. Dimethylformamid und Dimethylacetamid) und Sulfoxide (z.B. Dimethylsulfoxid).

Beispiele für die Streckmittel oder Träger umfassen anorganische Pulver, beispielsweise Löschkalk, Magnesiumkalk, Gips, Calciumcarbonat, Siliciumdioxid, Perlit, Bimsstein, Calcit, Diatomeenerde, amorphes Siliciumdioxid, Aluminiumoxid, Zeolithe und Tonminerale (z.B. Pyrophyllit, Talkum, Montmorillonit, Beidellit, Vermikulit, Kaolinit und Glimmer), pflanzliche Pulver wie beispielsweise Getreidepulver, Stärkearten, verarbeitete Stärkearten, Zucker, Glucose und zerkleinerte Stengel von Pflanzen, sowie Pulver aus synthetischen Harzen wie Phenol-Harzen, Harnstoff-Harzen und Vinylchlorid-Harzen.

Beispiele für oberflächenaktive Mittel umfassen anionische oberflächenaktive Mittel wie Alkylsulfonsäureester (z.B. Natriumlaurylsulfat), Arylsulfonsäuren (z.B. Alkylarylsulfonsäure-Salze und Natriumalkylnaphthalinsulfonate), Bernsteinsäure-Salze und Salze von Schwefelsäureestern von Polyethylenglycol-alkylarylethern, kationische oberflächenaktive Mittel wie Alkylamine (z.B. Laurylamin, Stearyltrimethylammoniumchlorid und Alkyldimethylbenzylammoniumchlorid) und Polyoxyethylenalkylamine, nicht-ionische oberflächenaktive Mittel wie Polyoxyethylenglycolether (z.B. Polyoxyethylenalkylarylether und deren Kondensationsprodukte), Polyoxyethylenglycolester (z.B. Polyoxyethylenfettsäureester) und Ester mehrwertiger Alkohole (z.B. Polyoxyethylensorbitan-monolaurat) sowie amphotere oberflächenaktive Mittel.

Beispiele für andere Hilfsstoffe umfassen Stabilisatoren, Haftmittel (z.B. landwirtschaftliche Seifen, Casein-Kalk, Natriumalginat, Polyvinylalkohol, Haftmittel vom Vinylacetat-Typ und Acryl-Haftmittel), Aerosol-Treibmittel (z.B. Trichlorofluoromethan, Dichlorofluoromethan, 1,2,2-Trichloro-1,1,2-trifluoroethan, Chlorbenzol, verflüssigtes Erdgas und niedere Ether), die Verbrennung steuernde Mittel für Räucherpräparate (z.B. Nitrite, Zink-Pulver und Dicyandiamid), Sauerstoff abgebende Mittel (z.B. Chlorate und Bichromate), Dispersions-Stabilisatoren /̄ z.B. Casein, Tragant, Carboxymethylcellulose (CMC) und Polyvinylalkohol (PVA) _7 und synergistische Mittel.

Die Verbindungen gemäß der vorliegenden Erfindung können mittels allgemein bei der Herstellung von Agrochemikalien angewandter Methoden zu verschiedenartigen Präparaten formuliert werden. Zu Beispielen für solche Anwendungsformen zählen emulgierbare Konzentrate, Öl-Präparate, benetzbare Pulver, lösliche Pulver, Suspensionen, Stäubemittel, Granulat, Pulverpräparate, Räuchermittel, Tabletten, Aerosole, Pasten und Kapseln.

Das landwirtschaftliche Fungizid gemäß der vorliegenden Erfindung kann etwa 0,1 bis etwa 95 Gew.-%, vorzugsweise etwa 0,5 bis etwa 90 Gew.-% des vorerwähnten aktiven Wirkstoffes enthalten.

Für den praktischen Gebrauch beträgt die geeignete Menge der aktiven Verbindung in den vorgenannten Mitteln

in ihren verschiedenen Formen und gebrauchsfertigen Präparaten im allgemeinen etwa 0,0001 bis etwa 20 Gew.-%, vorzugsweise etwa 0,005 bis etwa 10 Gew.-%.

Der Gehalt des Wirkstoffs kann in geeigneter Weise je nach der Form des Präparats, dem Verfahren, Zweck, der Zeit und dem Ort seiner Anwendung, dem Zustand des Auftretens einer Pflanzenkrankheit etc. variiert werden.

Erforderlichenfalls können die Verbindungen gemäß der vorliegenden Erfindung weiterhin auch in Kombination mit anderen Agrochemikalien verwendet werden, beispielsweise mit Insektiziden, anderen Fungiziden, Mitiziden, Nematiziden, Anti-Virus-Mitteln, selektiven Herbiziden, Pflanzen-Wachstumsregulatoren und Lockstoffen /¯z.B. Organophosphat-Verbindungen, Carbamat-Verbindungen, Dithio(oder thiol)carbamat-Verbindungen, organischen Chlor-Verbindungen, Dinitro-Verbindungen, organischen Schwefel- oder Metall-Verbindungen, Antibiotika, substituierten Diphenylether-Verbindungen, Harnstoff-Verbindungen und Triazin-Verbindungen_7 und/oder Düngemitteln.

Die den im Vorstehenden bezeichneten Wirkstoff enthaltenden verschiedenartigen Mittel und gebrauchsfertigen Präparate können mittels verschiedener Verfahren zur Anwendung gebracht werden, wie sie allgemein für das Aufbringen von Agrochemikalien gebräuchlich sind, beispielsweise durch Sprühen (Versprühen von Flüssigkeiten, Vernebeln, Zerstäuben, Stäuben, Streuen von Granulat, Wasser-Oberflächenbehandlung, Gießen etc.), Räuchern, Bodenbehandlung (Vermischen mit dem Boden, Streuen, Bedampfen, Gießen etc.), Oberflächenbehandlung

(Beschichten, Bändern, Bestäuben, Bedecken etc.) und Eintauchen. Sie können auch mittels des sogenannten Ultra-Low-Volume-Sprühverfahrens aufgebracht werden. Nach diesem Verfahren kann der Wirkstoff sogar in einer Konzentration von 100 % zur Anwendung gelangen.

Die Aufwandmengen pro Flächeneinheit betragen beispielsweise etwa 0,03 bis etwa 10 kg/ha, vorzugsweise etwa 0,3 bis etwa 6 kg/ha. In besonders gelagerten Fällen können oder sollten sogar die Aufwandmengen außerhalb des angegebenen Bereichs liegen.

Gemäß der vorliegenden Erfindung kann ein fungizides Mittel für die Landwirtschaft verfügbar gemacht werden, das eine Verbindung der allgemeinen Formel (I) als Wirkstoff und ein Verdünnungsmittel (ein Lösungsmittel und/oder ein Streckmittel und/oder einen Träger) und/oder ein oberflächenaktives Mittel und, sofern weiter erforderlich, einen Stabilisator, ein Haftmittel, ein synergistisches Mittel etc. enthält.

Die vorliegende Erfindung stellt ebenfalls ein Verfahren zur Bekämpfung von Pflanzenkrankheiten zur Verfügung, bei dem auf pathogene Pilze und/oder deren Lebensraum und den Ort des Auftretens der Erkrankungen die Verbindung der Formel (I) allein oder im Gemisch mit einem Verdünnungsmittel (einem Lösungsmittel und/oder einem Streckmittel und/oder einem Träger) und/oder einem oberflächenaktiven Mittel und, sofern weiter erforderlich, einem Stabilisator, einem Haftmittel, einem synergistischen Mittel etc. aufgebracht wird.

Die folgenden Beispiele erläutern die vorliegende Erfindung im einzelnen. Es sei jedoch darauf hingewiesen, daß die vorliegende Erfindung nicht allein auf diese speziellen Beispiele beschränkt ist.

Beispiel 1

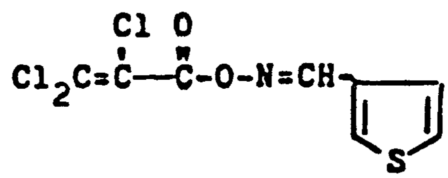

(Verbindung Nr. 1)

Thiophen-3-aldoxim (12,7 g) und Triethylamin (10,1 g) wurden in Toluol (300 ml) gelöst, und zu der erhaltenen Lösung wurde bei weniger als 20°C eine Lösung von Trichloroacryloylchlorid (19,4 g) in Toluol (100 ml) hinzugegeben. Die Reaktionspartner wurden 2 h bei 40°C zur Umsetzung gebracht. Das entstandene Triethylamin-hydrochlorid wurde durch Filtration abgetrennt, und die organische Schicht wurde mit Wasser gewaschen und getrocknet. Das Toluol wurde abgedampft, und der Rückstand wurde aus Isopropylether/n-Hexan umkristallisiert, wonach Thiophen-3-aldoxim-trichloroacrylat (25,8 g) in Form blaßgelber Kristalle erhalten wurde. Schmelzpunkt 93-95°C.

In ähnlicher Weise wie in Beispiel 1 synthetisierte Verbindungen der vorliegenden Erfindung sind in Tabelle 1 aufgeführt.

Tabelle 1

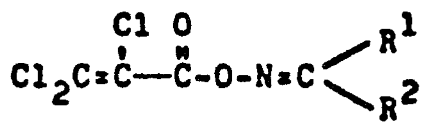

| Verbindung Nr. | $R^1$ | $R^2$ | Physikalische Konstante |
|---|---|---|---|
| 2 | H | 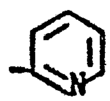 | Schmp. 74 - 74.5 °C |
| 3 | H | 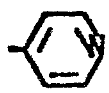 | Schmp. 107 - 108 °C |
| 4 | H | 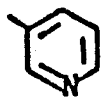 | Schmp. 101 - 103 °C |
| 5 | H | 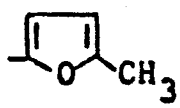 | Schmp. 63 °C |
| 6 | $-CH_3$ | 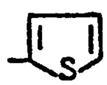 | Schmp. 77 - 78 °C |
| 7 | $-CH_3$ | 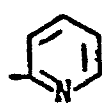 | Schmp. 53 - 54 °C |
| 8 | $-CH_3$ | 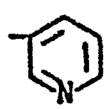 | Schmp. 82 - 84 °C |
| 9 | $-CH_3$ | 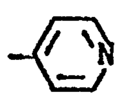 | Schmp. 77 - 78 °C |
| 10 | 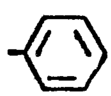 | 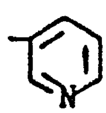 | Schmp. 125 - 126 °C |
| 11 | 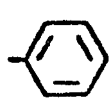 | 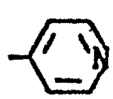 | Schmp. 165 - 167 °C |

Tabelle 1 - Fortsetzung

| Verbindung Nr. | $R^1$ | $R^2$ | Physikalische Konstante |
|---|---|---|---|
| 12 | 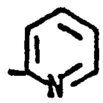 | 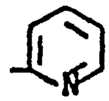 | Schmp. 123 - 126 °C |
| 13 | 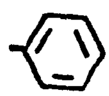 | 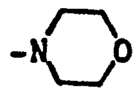 | $\nu_{C=O}$(Nujol)1740cm$^{-1}$ |
| 14 | 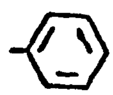 | 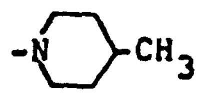 | $\nu_{C=O}$(Nujcl)1740cm$^{-1}$ |
| 15 | $-CH_3$ | 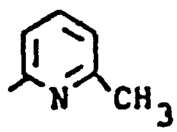 | Schmp. 74 - 75.5 °C |

Beispiel 2 (Benetzbares Pulver)

15 Teile der Verbindung Nr. 4 der Erfindung, 80 Teile eines Gemisches (1:5) aus Weißruß (wasserhaltigem, amorphen Kieselsäure-Pulver) und Tonpulver, 2 Teile Natriumalkylbenzolsulfonat und 3 Teile Natriumalkylnaphthalinsulfonat/Formaldehyd-Kondensat werden pulverisiert und zu einem benetzbaren Pulver vermischt. Dieses wird mit Wasser verdünnt und auf einen pathogenen Pilz und/oder seinen Lebensraum und den Ort, an dem eine Pflanzenkrankheit auftritt, aufgesprüht.

Beispiel 3 (Emulgierbares Konzentrat)

30 Teile der Verbindung Nr. 13 der Erfindung, 55 Teile Xylol, 8 Teile Polyoxyethylen-alkylphenylether und 7 Teile Calciumalkylbenzolsulfonat werden unter Rühren miteinander vermischt, wodurch ein emulgierbares Konzentrat hergestellt wird. Dieses wird mit Wasser verdünnt und auf einen pathogenen Pilz und/oder seinen Lebensraum und den Ort, an dem eine Pflanzenkrankheit auftritt, aufgesprüht.

Beispiel 4 (Stäubemittel)

2 Teile der Verbindung Nr. 14 der Erfindung und 98 Teile Tonpulver werden pulverisiert und gemischt, wodurch ein Stäubemittel hergestellt wird. Dieses wird über einem pathogenen Pilz und/oder seinen Lebensraum und den Ort, an dem eine Pflanzenkrankheit auftritt, ausgestreut.

Beispiel 5 (Stäubemittel)

Die Verbindung Nr. 8 der Erfindung (1,5 Teile), 0,5 Teile Isopropylhydrogenphosphat und 98 Teile Tonpulver werden pulverisiert und gemischt, wodurch ein Stäubemittel hergestellt wird. Dieses wird über einem pathogenen Pilz und/oder seinem Lebensraum und dem Ort, an dem eine Pflanzenkrankheit auftritt, ausgestreut.

Beispiel 6 (Granulat)

Wasser (25 Teile) wird zu einer Mischung aus 10 Teilen der Verbindung Nr. 5 der Erfindung, 30 Teilen Bentonit

(Montmorillonit), 58 Teilen Talkum und 2 Teilen Ligninsulfonat zugesetzt, und das Gemisch wird gut geknetet. Die Mischung wird mittels eines Extruder-Granulators zu einem Granulat mit einer Korngröße von 0,43 bis 2,0 mm (10 bis 40 mesh) verarbeitet und bei 40°C bis 50°C getrocknet, wodurch ein Granulat hergestellt wird. Das Granulat wird über einem pathogenen Pilz und/oder seinem Lebensraum und dem Ort, an dem eine Pflanzenkrankheit auftritt, ausgestreut.

Beispiel 7

Test auf Wirksamkeit gegen die Brusone-Krankheit von Reis bei Wasseroberflächen-Anwendung:

Herstellung einer Test-Verbindung:

| | |
|---|---|
| Wirkstoff: | 50 Gew.-Teile; |
| Träger: | 45 Gew.-Teile einer Mischung (1 : 5) aus Diatomeenerde und Kaolin; |
| Emulgator: | 5 Gew.-Teile Polyoxyethylenalkylphenylether. |

Die aktive Verbindung, der Träger und der Emulgator wurden in den angegebenen Mengen miteinander vermischt und pulverisiert, wodurch ein benetzbares Pulver gebildet wurde. Eine vorher festgelegte Menge des benetzbaren Pulvers wurde mit Wasser verdünnt, wodurch ein Test-Präparat hergestellt wurde.

Test-Verfahren

Reispflanzen (Varietät: Asahi) wurden in überfluteten Porzellan-Töpfen mit einem Durchmesser von 12 cm, jeweils drei Setzlinge pro Topf, kultiviert. Im Stadium

des Austreibens der Reispflanzen wurde die wie oben auf eine vorher festgelegte Konzentration gebrachte Test-Verbindung in den angegebenen Dosierungen auf die Wasseroberfläche gegossen, so daß sie nicht unmittelbar mit den oberirdischen Teilen der Reispflanzen in Berührung kam. Vier Tage später wurde eine Suspension von Sporen des Brusone-Pilzes (Piricularia oryzae) auf die Reispflanzen gesprüht, um sie mit dem Pilz zu inokulieren. Die Pflanzen wurden 24 h in einem Inkubator bei 23-25°C und einer relativen Luftfeuchtigkeit von 100 % gehalten. Danach wurden sie in ein auf 20-28°C gehaltenes Glas-Gewächshaus überführt. Sieben Tage nach der Beimpfung wurden die Pflanzen untersucht und aufgrund des folgenden Bewertungsmaßstabs beurteilt. Der Bekämpfungs-Index (%) wurde berechnet.

| Grad der Erkrankung | Fläche der Verletzungen (%) |
|---|---|
| 0 | 0 |
| 0,5 | 2 oder weniger |
| 1 | 3 bis 5 |
| 2 | 6 bis 10 |
| 3 | 11 bis 20 |
| 4 | 21 bis 40 |
| 5 | 41 oder mehr |

$$\text{Bekämpfungs-Index (\%)} = \frac{\text{Grad der Erkrankung einer unbehandelten Fläche} - \text{Grad der Erkrankung einer behandelten Fläche}}{\text{Grad der Erkrankung einer unbehandelten Fläche}} \times 100$$

Die Ergebnisse sind in Tabelle 2 aufgeführt. Diese Ergebnisse wurden aus drei Töpfen pro Prüfung erhalten.

Tabelle 2

| Verbindung Nr. | Wirkstoff-Konzentration $g/m^2$ | Bekämpfungs-Index (%) | Phyto-toxizität |
|---|---|---|---|
| 1 | 0,2 | 100 | - |
| 3 | 0,2 | 100 | - |
| 4 | 0,2 | 100 | - |
| 5 | 0,2 | 100 | - |
| 9 | 0,2 | 100 | - |
| Vergleich: | | | |
| A | 0,51 | 80 | - |

Anmerkungen zu Tabelle 2:

(1) Das Zeichen "-" in der Spalte der Phytotoxizität gibt an, daß keine Phytotoxizität vorlag.

(2) A: Kitazin P (chemischer Name: S-Benzyl-O,O-diisopropylphosphorothioat), 17 % Granulat (im Handel erhältlich).

Beispiel 8

Test auf Wirksamkeit gegen die Brusone-Krankheit von Reis bei Anwendung auf Stengel und Laub:

Test-Verfahren

Reispflanzen (Varietät: Asahi) wurden in unglasierten Töpfen mit einem Durchmesser von 12 cm kultiviert. Im

3- bis 4-Blatt-Stadium wurde eine wie in Beispiel 7 hergestellte Verdünnung einer Test-Verbindung mit vorher festgelegter Konzentration in einer Menge von 50 ml auf jeweils drei Töpfe aufgesprüht. Am nächsten Tag wurde eine künstlich kultivierte Suspension von Sporen des Brusone-Pilzes (Piricularia oryzae) zweimal auf die Reispflanzen gesprüht. Die Pflanzen wurden zur Herbeiführung der Infektion in einer Kammer bei einer Temperatur von 25°C und einer relativen Luftfeuchtigkeit von 100 % gehalten. Die Pflanzen wurden sieben Tage nach der Beimpfung in gleicher Weise wie in Beispiel 7 untersucht, und der Bekämpfungs-Index (%) wurde berechnet.

Die Ergebnisse sind in Tabelle 3 dargestellt.

Tabelle 3

| Verbindung Nr. | Wirkstoff-Konzentration ppm | Bekämpfungs-Index (%) | Phyto-toxizität |
|---|---|---|---|
| 1 | 500 | 100 | - |
| 4 | 500 | 100 | - |
| 7 | 500 | 100 | - |
| 8 | 500 | 100 | - |
| 9 | 500 | 100 | - |
| 10 | 500 | 100 | - |
| 11 | 500 | 100 | - |
| 12 | 500 | 100 | - |
| 13 | 500 | 100 | - |
| 14 | 500 | 100 | - |
| 15 | 500 | 100 | - |
| Vergleich: A' | 500 | 78 | - |

Anmerkungen zu Tabelle 3:

(1) Die Bezeichnung der Phytotoxizität ist die gleiche wie in Tabelle 2.

(2) Vergleich: A': Kitazin P, 48 % emulgierbares Konzentrat (im Handel erhältlich).

Die soweit im einzelnen beschriebene vorliegende Erfindung wird wie folgt zusammengefaßt:

1) Trichloroacryloyloxim-Derivat der allgemeinen Formel

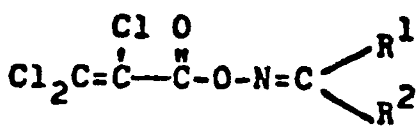

(I) ,

in der
$R^1$ und $R^2$ die oben angegebenen Bedeutungen haben.

2) Ein Verfahren zur Herstellung des Trichloroacryloyloxim-Derivats der Formel (I), bei dem ein Acylhalogenid oder -anhydrid der allgemeinen Formel

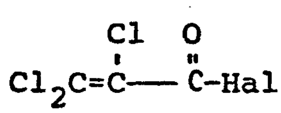 oder 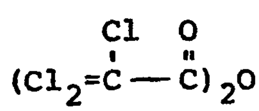 ,

in der Hal die angegebene Bedeutung hat, mit einem Oxim der allgemeinen Formel

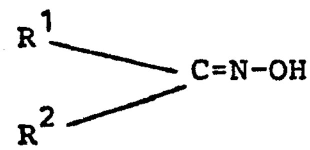

,

in der
$R^1$ und $R^2$ die oben angegebenen Bedeutungen haben, umgesetzt wird.

3) Ein das Trichloroacryloyloxim-Derivat der allgemeinen Formel (I) als Wirkstoff enthaltendes landwirtschaftliches Fungizid.

4) Ein Verfahren zur Bekämpfung einer Pflanzenkrankheit, bei dem eine Verbindung der allgemeinen Formel (I) entweder für sich allein oder in Form eines Gemisches mit einem Verdünnungsmittel (einem Lösungsmittel und/oder einem Streckmittel und/oder einem Träger) und/oder einem oberflächenaktiven Mittel und, falls weiterhin erforderlich, mit einem Stabilisator, einem Haftmittel und einem synergistischen Mittel zur Anwendung gebracht werden.

## Patentansprüche

1. Trichloroacryloyloxim-Derivate der Formel (I)

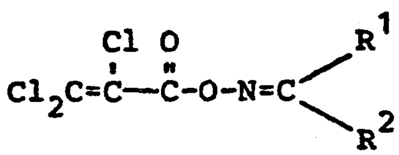

(I) ,

in der

$R^1$ ein Wasserstoff-Atom, eine niedere Alkyl-Gruppe, eine Phenyl-Gruppe oder eine 2-Pyridyl-Gruppe bezeichnet und

$R^2$ eine, gegebenenfalls durch Methyl substituierte, monocyclische heterocyclische Gruppe mit wenigstens einem Hetero-Atom bezeichnet.

2. Trichloroacryloyloxim-Derivate nach Anspruch 1, dadurch gekennzeichnet, daß in der Formel (I)

$R^1$ ein Wasserstoff-Atom, eine niedere Alkyl-Gruppe, eine Phenyl-Gruppe oder eine 2-Pyridyl-Gruppe bezeichnet und

$R^2$ eine, gegebenenfalls durch Methyl substituierte, monocyclische heterocyclische Gruppe mit wenigstens einem Sauerstoff-, Schwefel- oder Stickstoff-Atom bezeichnet.

Nit 170

3. Trichloroacryloyloxim-Derivate nach Anspruch 1, dadurch gekennzeichnet, daß in der Formel (I)
   $R^1$ Wasserstoff, Methyl, Ethyl, n-Propyl, iso-Propyl, n-, iso-, sec- oder tert-Butyl, Phenyl oder 2-Pyridyl bezeichnet und
   $R^2$ gegebenenfalls durch Methyl substituiertes Pyridyl, Thienyl, Furanyl, Morpholinyl oder Piperidinyl bezeichnet.

4. Verfahren zur Herstellung von Trichloroacryloyloxim-Derivaten der Formel (I)

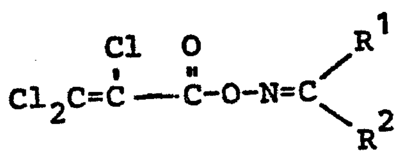

(I)

,

in der
$R^1$ ein Wasserstoff-Atom, eine niedere Alkyl-Gruppe, eine Phenyl-Gruppe oder eine 2-Pyridyl-Gruppe bezeichnet und
$R^2$ eine, gegebenenfalls durch Methyl substituierte, monocyclische heterocyclische Gruppe mit wenigstens einem Hetero-Atom bezeichnet,
dadurch gekennzeichnet, daß
a) ein Acylhalogenid der Formel (II)

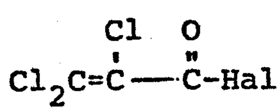

(II)

,

in der
Hal ein Halogen-Atom bezeichnet,
mit einem Oxim der Formel (III)

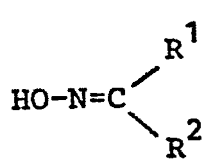

(III)

,

in der
$R^1$ und $R^2$ die oben angegebenen Bedeutungen haben, umgesetzt wird, falls angebracht in einem Lösungsmittel oder Verdünnungsmittel und falls angebracht in Gegenwart eines säurebindenden Mittels, oder

b) ein Acylanhydrid der Formel (IV)

$$(Cl_2C{=}\overset{Cl}{\overset{|}{C}}{-}\overset{O}{\overset{\|}{C}}{-})_2O \qquad (IV)$$

mit einem Oxim der Formel (III), falls angebracht in einem Lösungsmittel oder Verdünnungsmittel, umgesetzt wird.

5. Mittel zur Bekämpfung von Fungi, dadurch gekennzeichnet, daß sie wenigstens ein Trichloroacryloyloxim-Derivat der Formel (I) enthalten.

6. Verwendung von Trichloroacryloyloxim-Derivaten der Formel (I) zur Bekämpfung von Fungi.

7. Verfahren zur Bekämpfung von Fungi, dadurch gekennzeichnet, daß man Trichloroacryloyloxim-Derivate der Formel (I) auf Fungi und/oder deren Umgebung einwirken läßt.

8. Verfahren zur Herstellung von Mitteln zur Bekämpfung von Fungi, dadurch gekennzeichnet, daß Trichloroacryloyloxim-Derivate der Formel (I) mit Streckmitteln und/oder oberflächenaktiven Mitteln vermischt werden.